# EUROPEAN PATENT SPECIFICATION

(11) **EP 4 195 924 B1**
(45) Date of publication and mention of the grant of the patent: **25.06.2025**
(21) Application number: 21762083.0
(22) Date of filing: 17.08.2021
(51) Int. Cl.: A01N 25/30, A01N 43/90, A01N 47/44, A01P 1/00, A61K 8/41, A61K 8/43, A61K 8/49

(54) **DISINFECTANT COMPOSITION**
DESINFEKTIONSMITTELZUSAMMENSETZUNG
COMPOSITION DÉSINFECTANTE

(30) Priority: 17.08.2020 GB 202012797
(43) Date of publication of application: 21.06.2023
(73) Proprietor: Helperby Therapeutics Limited, London, Greater London WC2A 3LH (GB)
(72) Inventor: BRIDGE, George, Blackpool Lancashire FY1 2AE (GB); COATES, Professor Anthony Robert Milnes, London EC2A 3LH (GB); PELHAM, Paul, Blackpool Lancashire FY1 2AE (GB)
(74) Representative: HGF
(86) International application number: PCT/GB2021/052133
(87) International publication number: WO 2022/038351

(56) References cited:
- WO-A1-2008/056151
- WO-A1-2012/017215
- WO-A1-2016/166515
- WO-A1-2017/222965
- WO-A1-2018/033718
- WO-A2-2010/010345
- MOOSAVI MAHDIEH-SADAT ET AL: "Antiviral mouthwashes: possible benefit for COVID-19 with evidence-based approach", JOURNAL OF ORAL MICROBIOLOGY, vol. 12, no. 1, 1 January 2020 (2020-01-01), pages 1794363, XP055828110, Retrieved from the Internet <URL:http://socendoconcepcion.cl/descargas/covid19/articulos/Antiviral%20mouthwashes%20possible%20benefit%20for%20COVID%2019%20with%20evidence%20based%20approach.pdf> DOI: 10.1080/20002297.2020.1794363
- SHIRAI JUNSUKE ET AL: "Effects of Chlorine, Iodine, and Quaternary Ammonium Compound Disinfectants on Several Exotic Disease Viruses.", JOURNAL OF VETERINARY MEDICAL SCIENCE, vol. 62, no. 1, 1 January 2000 (2000-01-01), JP, pages 85 - 92, XP055857635, ISSN: 0916-7250, Retrieved from the Internet <URL:https://www.jstage.jst.go.jp/article/jvms/62/1/62_1_85/_pdf/-char/en> DOI: 10.1292/jvms.62.85
- Y. HU ET AL: "Enhancement by novel anti-methicillin-resistant Staphylococcus aureus compound HT61 of the activity of neomycin, gentamicin, mupirocin and chlorhexidine: in vitro and in vivo studies", JOURNAL OF ANTIMICROBIAL CHEMOTHERAPY., vol. 68, no. 2, 1 February 2013 (2013-02-01), GB, pages 374 - 384, XP055253047, ISSN: 0305-7453, DOI: 10.1093/jac/dks384
- HUBBARD ALASDAIR T. M. ET AL: "Mechanism of Action of a Membrane-Active Quinoline-Based Antimicrobial on Natural and Model Bacterial Membranes", BIOCHEMISTRY, vol. 56, no. 8, 28 February 2017 (2017-02-28), pages 1163 - 1174, XP055857022, ISSN: 0006-2960, Retrieved from the Internet <URL:https://pubs.acs.org/doi/pdf/10.1021/acs.biochem.6b01135> DOI: 10.1021/acs.biochem.6b01135
- HUBBARD ALASDAIR TM ET AL: "Comparing the action of HT61 and chlorhexidine on natural and model Staphylococcus aureus membranes", THE JOURNAL OF ANTIBIOTICS, vol. 70, no. 10, 1 October 2017 (2017-10-01), London, pages 1020 - 1025, XP055857021, ISSN: 0021-8820, Retrieved from the Internet <URL:https://www.nature.com/articles/ja201790.pdf> DOI: 10.1038/ja.2017.90

## Description

### TECHNICAL FIELD

This invention relates to a disinfectant composition and its use in a consumer product, as well as a method of disinfecting an article or surface with said composition or consumer product.

### BACKGROUND

Skin disinfecting or sanitizing compositions have become increasingly popular in the health care industry, as well as with the general public, for providing antimicrobial effectiveness. This is especially the case following the outbreak of the disease COVID-19 caused by the new Coronavirus 2019-nCoV [officially designated as severe acute respiratory syndrome-related Coronavirus SARS-CoV 2].

Coronaviruses (CoVs) are relatively large viruses containing a single-stranded positive-sense RNA genome encapsulated within a membrane envelope. The viral membrane is studded with glycoprotein spikes that give Coronaviruses their crown-like appearance. There are four classes of Coronaviruses designated as alpha, beta, gamma, and delta. The beta-Coronavirus class includes severe acute respiratory syndrome (SARS) virus (SARS-CoV), Middle East respiratory syndrome (MERS) virus (MERS-CoV), and the COVID-19 causative agent SARS-CoV-2. Similar to SARS-CoV and MERS-CoV, SARS-CoV-2 attacks the lower respiratory system to cause viral pneumonia; it may also affect the gastrointestinal system, heart, kidney, liver, and central nervous system leading to multiple organ failure.

The beta-Coronavirus genome encodes several structural proteins, including the glycosylated spike (S) protein that functions as a major inducer of host immune responses. This S protein mediates host cell invasion by both SARS-CoV and SARS-CoV-2 via binding to a receptor protein called angiotensin-converting enzyme 2 (ACE2) located on the surface membrane of host cells. According to a study published in 2015 by Baez-Santos (Antiviral Research (2015), 115, 21-28), this invasion process requires S protein priming which is facilitated by the host cell-produced serine protease TMPRSS211. In addition, the viral genome encodes several non-structural proteins including RNA-dependent RNA polymerase (RdRp), Coronavirus main protease (3CLpro), and papain-like protease (PLpro). Upon entrance to the host cells, the viral genome is released as a single-stranded positive RNA. Subsequently, it is translated into viral polyproteins using host cell protein translation machinery, which are then cleaved into effector proteins by viral proteinases 3CLpro and PLpro. PLpro also behaves as a deubiquitinase that may deubiquinate certain host cell proteins, including interferon factor 3 and NF- 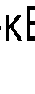 B, resulting in immune suppression (Lee et al., Journal of Molecular Biology (2005), 353 (5), 1137-1151). RdRp synthesizes a full-length negative-strand RNA template to be used by RdRp to make more viral genomic RNA.

The interaction between viral S protein and ACE2 on the host cell surface is of significant interest since it initiates the infection process. Cryo-EM structure analysis has revealed that the binding affinity of SARS-CoV-2 S protein to ACE2 is about 10-20 times higher than that of SARS-CoV S protein (Wrapp et al., Science 2020 Mar 13, 367 (6483);1260-1263; Lu et al., Lancet, 2020 Feb 22;395(10224):565-574). It is speculated that this may contribute to the reported higher transmissibility and contagiousness of SARS-CoV-2 as compared to SARS-CoV.

Alongside the development of therapeutics to target SARS-CoV-2, there has been significant interest in compositions that can help prevent the transmission of the virus and other pathogens by way of the skin. Specifically compositions that can be readily applied to surfaces and articles, and act efficiently on application thereto in order to prevent the spread of the infection. This is a particularly challenging task since SAR-Cov-2, like the other known Coronaviruses, is an enveloped virus and enveloped viruses have a unique resistance to chemicals and environment conditions. The desired formulations for controlling and killing enveloped viruses such as Coronaviruses may be disinfectants and in particular may be disinfectants showing antibacterial activity, virucidal activity and/or sporicidal activity.

Antibacterial activity may be measured by the BS EN 1500 Hygienic Handrub Method; any reference herein to BS EN 1500 is to BS EN 1500:2013. This method is a European Standard test method that evaluates the efficacy of a hygienic handrub through measuring the number of viable bacteria remaining on the fingertips after contamination and handrub exposure. A handrub is defined as a treatment that involves rubbing the hands without the addition of water. This method specifically simulates conditions for establishing if a hygienic handrub decreases the release of transient flora from the hands.

Virucidal activity may be measured by the BS EN 14476 Chemical disinfectants and antiseptics method; a quantitative suspension test for the evaluation of virucidal activity in the medical area. Any reference herein to BS EN 14476 is to BS EN 14476:2013+A2:2019. This method is a European Standard test method that sets out the minimum requirements for virucidal activity of chemical disinfectant and antiseptic products that form a homogeneous physically stable preparation when diluted with hard water or in the case of ready-to-use products, i.e., products that are not diluted when applied, with water. This European Standard applies to products that are used in the medical area in the fields of hygienic handrub, hygienic handwash, instrument disinfection by immersion, surface disinfection by wiping, spraying, flooding or other means and textile disinfection. This European Standard applies to areas and situations where disinfection is medically indicated. Such indications occur in patient care, for example: in hospitals, in community medical facilities, and in dental institutions; in clinics of schools, of kindergartens, and of nursing homes; and may occur in the workplace and in the home. It may also include services such as laundries and kitchens supplying products directly for the patients.

Sporicidal activity may be measured by the BS EN 17126 Chemical disinfectants and antiseptics method; a quantitative suspension test for the evaluation of sporicidal activity of chemical disinfectants in the medical area. Any reference herein to BS EN 17126 is to BS EN 17126:2018. This method is a European Standard test that sets out the minimum requirements for sporicidal activity of chemical disinfectants that form a homogeneous, physically stable preparation when diluted with hard water, or in the case of ready-to-use products, with water. This European Standard applies to products that are used in the medical area in the fields of instrument disinfection by immersion, and surface disinfection by wiping, spraying, flooding or other means. This European Standard applies to areas and situations where disinfection is medically indicated. Such indications occur in patient care, for example: in hospitals, in community medical facilities and in dental institutions; in clinics of schools, of kindergartens and of nursing homes; and may occur in the workplace and in the home. It may also include services such as laundries and kitchens supplying products directly for the patients.

Despite the significant interest and activity in this area, there is still a need for a disinfectant composition that meets at least one of the above-described European Standards and in particular, a composition that meets all three Standards and is effective at killing enveloped viruses such as Coronaviruses. There is also a need in the art for such compositions to have limited side effects, particularly when used as a handrub or the like. The increased frequency and duration of hand-washing and hand-sanitizing as a result of COVID-19 has led to a rise in skin irritation problems, especially because most hand sanitizers rely on the use of an alcoholic solvent.

The present invention is aimed at addressing these needs.

### SUMMARY

In one aspect the invention provides the use of 0.001% w/v to 0.5% w/v of 4-methyl-8-phenoxy-1-(2-phenylethyl)-2,3-dihydro-1H-pyrrolo[3,2-c]quinoline or a pharmaceutically acceptable salt and/or solvate thereof as an emollient in an aqueous disinfectant composition. Preferably the disinfectant composition is a virucidal, antibacterial or sporicidal disinfectant composition. More preferably the disinfectant composition is virucidal, antibacterial and sporicidal. The terms "virucidal", "antibacterial" and "sporicidal" are defined herein with reference to the above-mentioned European Standards.

In a second aspect the invention provides a disinfectant composition comprising:
(a) 0.001% w/v to 0.5% w/v of 4-methyl-8-phenoxy-1-(2-phenylethyl)-2,3-dihydro-1H-pyrrolo[3,2-c]quinoline or a pharmaceutically acceptable salt and/or solvate thereof;
(b) 0.001% w/v to 4% w/v of chlorhexidine or a pharmaceutically acceptable salt and/or solvate thereof; and
(c) 0.001% w/v to 2% w/v of a dialkyldimethylammonium chloride, wherein the alkyl group has 8 or more carbon atoms, and where the composition is formulated as a single solution in an aqueous solvent.

In various embodiments of the invention, the composition preferably comprises 0.001% w/v to 0.2% w/v of 4-methyl-8-phenoxy-1-(2-phenylethyl)-2,3-dihydro-1H-pyrrolo[3,2-c]quinoline or a pharmaceutically acceptable salt and/or solvate thereof, more preferably 0.01% w/v to 0.1% w/v of 4-methyl-8-phenoxy-1-(2-phenylethyl)-2,3-dihydro-1H-pyrrolo[3,2-c]quinoline or a pharmaceutically acceptable salt and/or solvate thereof. At such low concentrations, 4-methyl-8-phenoxy-1-(2-phenylethyl)-2,3-dihydro-1H-pyrrolo[3,2-c]quinoline - otherwise known as "HT61" - functions as an emollient. This is discussed and exemplified further herein. HT61 is a compound developed by the Applicant and its synthesis and characterisation are disclosed in WO 2007/054693.

In various embodiments of the invention, the composition preferably comprises 0.001% w/v to 2% w/v chlorhexidine or a pharmaceutically acceptable salt and/or solvate thereof more preferably 0.05% w/v to about 0.5% w/v chlorhexidine or a pharmaceutically acceptable salt and/or solvate thereof.

In various embodiments of the invention, the dialkyldimethylammonium chloride is selected from dioctyldimethylammonium chloride, octyldecyldimethylammonium chloride, and didecyldimethylammonium chloride, preferably the dialkyldimethylammonium chloride is didecyldimethylammonium chloride, otherwise known as DDAC. In various embodiments of the invention, the composition preferably comprises 0.001% w/v to 1% w/v of didecyldimethylammonium chloride, more preferably 0.01% w/v to 0.7% w/v of didecyldimethylammonium chloride.

In various embodiments of the invention, the formulation is biodegradeable. The term "biodegradeable" means that the disinfectant composition is capable of being decomposed by bacteria or other living organisms and thereby having minimal environmental impact.

In various embodiments of the invention the aqueous solvent is water.

In various embodiments of the invention the disinfectant composition has virucidal activity as measured by BS EN 14476. The virucidal activity of the composition may specifically be against a Picornavirus, a Poxvirus, a Coronavirus or a combination thereof, preferably the virucidal activity is against Poliovirus, a Poxvirus such as Vaccinia virus, a Coronavirus or a combination thereof. More preferably the virucidal activity as measured by BS **EN** 14476 is against a Coronavirus.

In various embodiments of the invention the disinfectant composition has antibacterial activity as measured by BS EN 1500. In various embodiments of the invention the disinfectant composition is a sporicidal disinfectant composition, preferably the composition has sporicidal activity as measured by BS EN 17126.

In a third aspect the invention provides a consumer product comprising the disinfectant composition described herein. The consumer product may, for example, comprise at least one of a disinfectant spray, hand sanitizer solution or gel, antiseptic wipe, or antiseptic patch.

In a fourth aspect the invention provides a method of disinfecting an article or surface comprising treating said article or surface with the disinfectant composition described herein or the consumer product described herein.

These aspects and embodiments are set out in the appended independent and dependent claims. The foregoing and other objects, features, and advantages of the invention will appear more fully hereinafter from a consideration of the detailed description that follows.

For ease of reference, these and further aspects of the present disclosure are now discussed under appropriate section headings. However, the teachings under each section are not necessarily limited to each particular section.

### DETAILED DESCRIPTION

As used in this specification and the claims, the singular forms "a," "an," and "the" include plural referents unless the context clearly dictates otherwise. Reference to "dry weight percent" or "dry weight basis" refers to weight on the basis of dry ingredients (i.e., all ingredients except water). Reference to "wet weight" refers to the weight of the composition including water or other aqueous solvent. Unless otherwise indicated, reference to "weight percent" (or "% by weight") of a composition reflects the total wet weight of the composition (i.e., including water or other aqueous solvent).

In this specification, unless otherwise stated, the term "about" modifying the quantity of an ingredient refers to variation in the numerical quantity that can occur, for example, through typical measuring and liquid handling procedures used for making concentrates or solutions in the real world; through inadvertent error in these procedures; through differences in the manufacture, source, or purity of the ingredients employed, or to carry out the methods; and the like. The term "about" also encompasses amounts that differ due to different equilibrium conditions for a composition resulting from a particular initial mixture. Whether or not modified by the term "about", the claims include equivalents to the quantities.

The ranges provided herein provide exemplary amounts of each of the components. Each of these ranges may be taken alone or combined with one or more other component ranges.

The general inventive concept of the present disclosure is centred on using HT61 as an emollient in an aqueous disinfectant composition. HT61 is disclosed in WO 2007054693 A1 as an antimicrobial compound. Example 1(m) of WO 2007054693 A1 includes the preparation of the chloride salt along with an ¹H-NMR characterisation. HT61 is, however, used at a higher concentration than the present invention, namely 2.0 wt% (see Example 10), and in a formulation tested for biological activity against log-phase and stationary-phase bacteria which is not an aqueous disinfectant composition. There is no teaching in WO 2007054693 A1 that HT61 has any function at much lower concentrations, namely 0.001% w/v to 0.5% w/v, and no indication that the compound would perform well in an aqueous disinfectant composition as discussed herein.

Emollients help support the skin's barrier; they can create a protective layer on top of the skin and help prevent moisture escaping. They can also attract moisture into the top layer of the skin and retain it in order to rehydrate the skin. The function of an emollient thus includes softening, moisturizing, lubricating, protecting, film-forming, conditioning, solubilizing and dispersing. The emollient properties of HT61 are demonstrated and discussed further in the Examples. These properties are advantageous in the field of aqueous disinfectants for topical use because of the skin irritation and dermatitis problems discussed above.

More specifically, the inventive concept focusses on using HT61 as an emollient in a aqueous disinfectant composition which is formulated as a single solution and thus straightforward to manufacture. This disinfectant composition includes a defined amount of chlorhexidine and a defined amount of a dialkyldimethylammonium chloride and advantageously shows antibacterial, virucidal and/or sporicidal activity in one or more of the European Standard Tests discussed above.

Chlorhexidine is a commercially available antiseptic compound. It is sold in Europe under a variety of trade names for various therapeutic indications. For example, Corsodyl^{®} is a mouthwash comprising chlorhexidine digluconate (0.2% w/v) which is indicated for the inhibition of the formation of dental plaque, as an aid in the treatment and prevention of gingivitis, and in the maintenance of oral hygiene.

When used as a disinfectant and particularly a skin disinfectant, chlorhexidine is, however, typically used in an alcoholic solvent. Bactiseptic orange by Vesismin Health is, for example, an antiseptic formulated with a solution of 2% chlorhexidine and 70% isopropyl alcohol. Similarly RU 2 724 581 C1 discloses an antiseptic disinfectant including *inter alia* 40-50% n-propyl alcohol, 10-30% isopropyl alcohol and 0.4-0.6% chlorhexidine bigluconate. The use of such high levels of alcohol is not, however, desirable because there is a risk of substance abuse by consumers, and side-effects from the use of such products on the skin such as skin irritation and dermatitis.

Dialkyldimethylammonium chlorides with the alkyl group having 8 or more carbon atoms includes didecyldimethylammonium chloride or DDAC which is a commercially available antiseptic or disinfectant that is used in various biocidal applications. DDAC is, like chlorhexidine, typically used in an alcoholic solvent. K-CARE^{®} DDAC80 for example, contains up to 20% ethanol along with 78-82% DDAC.

WO 2012/017215 A1 and Y. Hu et al. (Journal of Antimicrobial Chemotherapy, 2013, 68, 374-384) disclose the use of HT61 with (*inter alia*) chlorhexidine for the prevention and/or treatment of microbial infections. WO 2018/033718 A1, WO 2017/222965 A1 and WO 2010/010345 A2 disclose compositions comprising chlorhexidine and dialkyldimethylammonium compounds.

In the present invention the combination of 0.001% w/v to 0.5% w/v of HT61 or a pharmaceutically acceptable salt and/or solvate thereof, 0.001% to 4% chlorhexidine or a pharmaceutically acceptable salt and/or solvate thereof, and 0.001% w/v to 2% w/v of a dialkyldimethylammonium chloride such as DDAC can be formulated as a single solution in an aqueous solvent and advantageously provides activity against enveloped viruses. This activity may be antibacterial, virucidal and/or sporicidal, and involve controlling and killing the virus.

The terms "disinfection" and "disinfectant" as used herein have the meaning understood by those skilled in the art of medicinal practice. In particular, the killing of - theoretically all - pathogenic germs harmful to human beings. The activity of the disinfectant composition can be measured by one or more of the European Standard Test Methods described above: BS EN 14476, BS EN 1500 and BS EN 17126. Each of these test methods is incorporated herein by reference. As explained above, each of these test methods is a European test standard; this means that the test has been approved by The European Committee for Standardization (CEN).

The BS EN14476 standard requires that a chemical disinfectant achieve at least a 4.0 log reduction in virus titer for one or more defined microorganisms. For surface disinfection, these microorganisms include Poliovirus (Poliovirus type 1, LSc 2ab), Norovirus (Murine norovirus, strain S99 Berlin) and Adenovirus (Adenovirus type 5, strain Adenoid 75, ATCC VR-5), and for a hygienic handrub and handwash, these microorganisms include Poliovirus (Poliovirus type 1, LSc 2ab) and Vaccinia virus (Modified vaccinia virus Ankara, ATCC VR- 1508, or vaccinia virus strain Elstree, ATCC VR-1549), the latter being indicative of virucidal activity against enveloped viruses and the former being indicative of virucidal activity against non-enveloped viruses.

All the viruses listed in the BS EN14476 standard are non-enveloped viruses except Vaccinia virus, and as such, are more resistant to chemical disinfectant. Poliovirus is the most resistant among the non-enveloped viruses listed, so efficacy and activity against Poliovirus is used to show virucidal activity in the present invention against non-enveloped viruses, whilst efficacy and activity against Vaccinia virus is used to show virucidal activity against enveloped viruses (including Coronaviruses).

As described in the BS EN14476 standard, in preparing for the suspension test, the test virus is added to an interfering substance in a suspension. The choice of interfering substance used in the test depends on the product claim. For hygienic handrub and handwash the interfering substance is 0.3 g/l bovine albumin (clean condition; hygienic handrub) or 3.0 g/l bovine albumin solution plus 3.0 ml/I erythrocytes (dirty condition; hygienic handwash). The test product or disinfectant is then added to the virus suspension for the duration of the exposure time at the temperature specified by the manufacturer (for example at 20°C or between 4°C and 30°C, with a contact time between 30 sec and 2 minutes or no longer than 5 minutes or 60 minutes). At the end of the exposure time, samples are retrieved and the activity of the test product is neutralised by dilution in ice-cold test medium. Serial dilutions are performed, and the dilutions are examined for viral infectivity. Unlike bacteria or fungus, most viruses are too small (ranging from 25 nm to 400 nm) for observation under a light microscope. The presence of viruses in a suspension before and after product exposure is therefore determined by inoculating live host cells with suspension samples. These cells are then observed after 7 days (depending on the cell type) for structural changes. If the test product had not been successful in inactivating test viruses before neutralization, they invade and damage the live cells to display cytopathic effect (CPE). These are the effects virologists look for when observing the cells under a light microscope.

BS EN 1500 is a European Standard test method that evaluates the efficacy of a hygienic handrub by measuring the number of viable bacteria remaining on the fingertips after contamination and handrub exposure. In this test, subjects with healthy hands are randomly assigned either a reference control product or the hygienic handrub under evaluation. Following an initial cleansing wash with soft soap to remove natural transient microorganisms, the subject's hands are dried thoroughly with a paper towel.

Typically a prepared pure culture of a non-pathogenic strain of *Escherichia coli* is used as the inoculum for this test method, such as the non-pathogenic *Escherichia coli* K12 (e.g. NCTC 10538). Subjects immerse their hands in a volume of the inoculum, up to the mid-carpals, for 5 seconds with their fingers spread apart. The hands are then allowed to air dry for 3 minutes, then the fingertips are sampled by rubbing them into a petri dish with sterile tryptic soy broth (TSB) for pre-values of viable bacteria present on the hands.

Immediately after pre-value sampling the hands are re-inoculated, then subjected to either the reference handrub procedure with propan-2-ol 60% (v/v), or the hygienic handrub being tested per instructions provided by the manufacturer. The fingertips are then sampled again for post-values in the same manner as the pre-values, this time with TSB containing a chemical neutralizer. The samples are then diluted appropriately and plated onto tryptic soy agar (TSA) medium, where they are allowed to incubate 18-24 hours at 36 ± 1°C, counted, then re-incubated for an additional 24 hours to detect any potential slow growing colonies.

The pre- and post-values recovered from the fingertips are evaluated against one another, resulting in a ratio called the reduction factor. The reduction factor provides a quantitative measure of the antimicrobial efficacy. This is determined by comparing average counts of viable microorganism colony forming units of the pre-values to the average counts after exposure to the hygienic handrub. The mean reduction of the release of the test organism E. *coli* K12 achieved by the hygienic handrub with the product under test should be at least not inferior to that achieved by the reference handrub (60% volume concentration of propan-2-ol).

BS EN 17126 is a European standard that describes a test method and the minimum requirements for evaluating the sporicidal activity of chemical products that form a homogenous and physically stable preparation when diluted with hard water or in the case of ready-to-use products, with water. In order to be characterised as sporicidal, a product shall demonstrate at least 4 decimal log reduction when tested in accordance with BS EN 17126.

For surface disinfection, the sporicidal activity in BS EN 17126 is tested against *Clostridium difficile* R027 (NCTC 13366) and/or *Bacillus subtilis* (ATCC 6633) and *Bacillus cereus* (CIP 105151) at a test temperature of 4°C to 30°C. A sample of the product as delivered and/or diluted with hard water (or water for ready to use products) is added to a test suspension of spores in a solution of an interfering substance (e.g. 0.3 g/l bovine albumin solution for clean conditions, 3.0 g/l bovine albumin solution plus 3.0 ml/I erythrocytes for dirty conditions). The mixture is maintained at the test temperature for a contact time of no longer than 15 minutes or no longer than 60 minutes depending on the practical conditions of the product. Products intended to disinfect surfaces that are likely to come into contact with a patient and/or medical staff and surfaces, which are frequently touched by different people, leading to the transmission of microorganisms to the patient, shall be tested with a contact time of maximum 15 minutes. Products for other surfaces may be tested with a contact time of maximum 60 minutes. At the end of the contact time, an aliquot is taken and the sporicidal action in this portion is immediately neutralized or suppressed by a validated method. The method of choice is dilution-neutralization. The number of surviving spores in each sample are determined and the reduction is calculated.

In various embodiments of the present invention, the disinfectant composition is effective in reducing the concentration or killing one or more enveloped or non-enveloped viruses. In various embodiments the disinfectant composition is effective in reducing the concentration or killing one or more non-enveloped virus such as Poliovirus and/or one or more enveloped virus such as Vaccinia virus, a Coronavirus and a combination thereof. In various embodiments the disinfectant composition is effective in reducing the concentration or killing one or more one or more enveloped virus such as Vaccinia virus, a Coronavirus and a combination thereof.

Viruses are microscopic, infectious organisms made generally of a nucleic acid (DNA/RNA) in a protein coating that infect the cells of a living organism. Viruses replicate by forcing the host cell to copy the virus' genetic material, and are categorized into two types based on the composition of their outer surface structure: enveloped viruses and non-enveloped viruses. Enveloped viruses have a protein coating, often called a capsid, which is further encased with an outer lipid membrane. The lipid membrane includes phospholipids, proteins and viral glycoproteins, where the glycoproteins allow the virus to identify and attach to receptor sites on the host's cell membranes. Conversely, non-enveloped viruses are surrounded only by the protein coating (capsid) which performs the task of attaching to host cells. Unlike enveloped viruses, non-enveloped viruses are typically resistant to harsh environmental conditions such as wide ranges of heat, dryness and acidic conditions. In fact, non-enveloped viruses can even survive and remain fully active/infectious in some disinfection processes. These characteristics thus make non-enveloped viruses particularly challenging to kill and control.

In various embodiments of the invention, the disinfectant composition is effective in reducing the concentration or killing one or more viruses selected from a Poliovirus, a Coronavirus or a combination thereof. In various embodiments the composition may be effective against enveloped viruses (as shown by activity against Vaccina virus). In various embodiments, the disinfectant composition is effective against a Poliovirus. In various embodiments, the disinfectant composition is effective against a Vaccinia virus. In various embodiments, the disinfectant composition is effective against a Coronavirus. In various embodiments the disinfectant composition is effective against a Poliovirus and a vaccina virus. In various embodiments, the disinfectant composition is effective against each of a Poliovirus, a Vaccinia virus and a Coronavirus. By the term "effective against" is meant decreasing the concentration of the virus.

Poliovirus is the causative agent of poliomyelitis (polio), a highly infectious viral disease which is transmitted horizontally. Three serotypes of Poliovirus have been identified: PV1, PV2 and PV3, each exhibiting a different capsid protein. Complications from polio include muscle weakness, paralysis and in some cases, death. As noted above, EN 14476 uses Poliovirus as a standard in measuring virucidal activity because it is a well-studied non-enveloped virus and similar in chemical resistance to a wide variety of other human viruses.

Coronaviruses are a group of related RNA viruses that cause diseases in mammals and birds. In humans these viruses cause respiratory tract infections that can range from mild to lethal. They are enveloped viruses with a positive-sense single-stranded RNA genome and a nucleocapsid of helical symmetry. In various embodiments of the present invention the disinfectant composition is effective against Coronaviruses, specifically feline Coronavirus according to the protocol of BS EN 14476. Feline Coronavirus (FCoV) is a positive-stranded RNA virus that infects cats worldwide. It is a Coronavirus of the species *AlphaCoronavirus 1* which includes canine Coronavirus and porcine transmissible gastroenteritis Coronavirus. Feline Coronavirus is well-known in the art and widely accepted as a surrogate for SARS CoV-2. Accordingly activity against FCoV can be extrapolated to activity against SARS CoV-2.

In various embodiments, the disinfectant composition reduces the concentration of enveloped viruses such as Vaccinia virus and/or a Coronavirus, and/or non-enveloped viruses such as the Poliovirus, by a statistically significant amount and in other embodiments, passes the EN 14476 standard for enveloped and/or non-enveloped virus concentration. The expression "statistically significant" means p<0.05 for a test composition vs a control that does not contain the active ingredient. The analysis is completed using 1) a T-test (a statistical examination of two population means); or 2) an analysis of variance (ANOVA) test when comparing two or more test articles vs controls.

In various embodiments the disinfectant composition shows antibacterial activity according to the BS EN 1500 protocol.

In various embodiments the disinfectant composition shows sporicidal activity according to the BS EN 17126 protocol. Such activity may involve reducing the amount of *Clostridium difficile* (*C.difficile*) on an article or surface.

As used herein the term *"pharmaceutically acceptable derivative"* means:
(a) pharmaceutically acceptable salts; and/or
(b) solvates (including hydrates).

Suitable acid addition salts include carboxylate salts (e.g. formate, acetate, trifluoroacetate, propionate, isobutyrate, heptanoate, decanoate, caprate, caprylate, stearate, acrylate, caproate, propiolate, ascorbate, citrate, glucuronate, glutamate, glycolate, α-hydroxybutyrate, lactate, tartrate, phenylacetate, mandelate, phenylpropionate, phenylbutyrate, benzoate, chlorobenzoate, methylbenzoate, hydroxybenzoate, methoxybenzoate, dinitrobenzoate, o-acetoxybenzoate, salicylate, nicotinate, isonicotinate, cinnamate, oxalate, malonate, succinate, suberate, sebacate, fumarate, malate, maleate, hydroxymaleate, hippurate, phthalate or terephthalate salts), halide salts (e.g. chloride, bromide or iodide salts), sulfonate salts (e.g. benzenesulfonate, methyl-, bromo- or chloro-benzenesulfonate, xylenesulfonate, methanesulfonate, ethanesulfonate, propanesulfonate, hydroxyethanesulfonate, 1- or 2-naphthalene-sulfonate or 1,5-naphthalenedisulfonate salts) or sulfate, pyrosulfate, bisulfate, sulfite, bisulfite, phosphate, monohydrogenphosphate, dihydrogenphosphate, metaphosphate, pyrophosphate or nitrate salts.

Suitable base salts include metal salts, e.g. sodium, calcium, and amine salts.

The invention also includes where appropriate all enantiomers and tautomers of the compounds. The skilled person will recognise compounds that possess optical properties (one or more chiral carbon atoms) or tautomeric characteristics. The corresponding enantiomers and/or tautomers may be isolated or prepared by methods known in the art.

Some of the compounds included in the compositions of the invention may exist as stereoisomers and/or geometric isomers - e.g. they may possess one or more asymmetric and/or geometric centres and so may exist in two or more stereoisomeric and/or geometric forms. The present invention contemplates the use of all the individual stereoisomers and geometric isomers of those inhibitor agents, and mixtures thereof. The terms used in the claims encompass these forms, provided said forms retain the appropriate functional activity (though not necessarily to the same degree).

The present invention also includes all suitable isotopic variations of the compounds or pharmaceutically acceptable salts thereof. An isotopic variation or a pharmaceutically acceptable salt thereof is defined as one in which at least one atom is replaced by an atom having the same atomic number but an atomic mass different from the atomic mass usually found in nature. Examples of isotopes that can be incorporated include isotopes of hydrogen, carbon, nitrogen, oxygen, phosphorus, sulphur, fluorine and chlorine such as 2H, 3H, 13C, 14C, 15N, 170, 180, 31P, 32P, 35S, 18F and 36Cl, respectively. Certain isotopic variations, for example, those in which a radioactive isotope such as 3H or 14C is incorporated, are useful in drug and/or substrate tissue distribution studies. Tritiated, i.e., 3H, and carbon-14, i.e., 14C, isotopes are particularly preferred for their ease of preparation and detectability. Further, substitution with isotopes such as deuterium, i.e., 2H, may afford certain therapeutic advantages resulting from greater metabolic stability, for example, increased in vivo half-life or reduced dosage requirements and hence may be preferred in some circumstances. Isotopic variations can generally be prepared by conventional procedures using appropriate isotopic variations of suitable reagents.

The compounds for use in the composition of the present invention, including the pharmaceutically acceptable derivatives thereof, are commercially available and/or can be prepared by synthesis methods known in the art.

4-methyl-8-phenoxy-1-(2-phenylethyl)-2,3-dihydro-1H-pyrrolo[3,2-c]quinoline and its pharmaceutically acceptable salts and/or solvates may, for example, be prepared according to the synthesis method set out in WO 2007/054693 A1 (e.g. in Example 1 and Example 9). For the avoidance of doubt the name 4-methyl-8-phenoxy-1-(2-phenylethyl)-2,3-dihydro-1H-pyrrolo[3,2-c]quinoline means a compound having the following chemical structure:

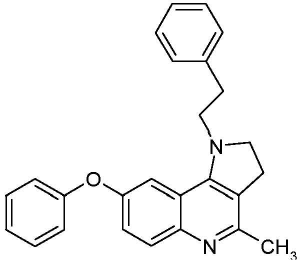

Preferred pharmaceutically acceptable derivatives of 4-methyl-8-phenoxy-1-(2-phenylethyl)-2,3-dihydro-1H-pyrrolo[3,2-c]quinoline include the hydrochloride and mesylate salts thereof. Chlorhexidine is a cationic polybiguanide also known as *N,N""*1,6-Hexanediylbis[*N'*-(4-chlorophenyl)(imidodicarbonimidic diamide)] with the chemical formula:

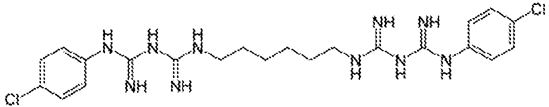

Chlorhexidine and its pharmaceutically acceptable salts and/or solvates are commercially available, for example from Sigma Aldrich Limited. Preferred pharmaceutically acceptable salts of chlorhexidine include the hydrochloride, dihydrochloride, diacetate, acetate, digluconate and gluconate salts thereof. Particularly preferred salts are the diacetate and digluconate salts, especially chlorhexidine digluconate.

The group of compounds falling within the formula: dialkyldimethylammonium chloride where the alkyl group has 8 or more carbon atoms are known in the art and may be prepared by known synthesis methods or obtained from commercial sources. The dialkyldimethylammonium chloride may be selected from: didecyldimethylammonium chloride, dioctyldimethylammonium chloride, distearyldimethylammonium chloride, octyldecyldimethylammonium chloride and dimethyldioctadecylammonium chloride. Preferably, the dialkyldimethylammonium chloride is selected from didecyldimethylammonium chloride, dioctyldimethylammonium chloride and octyldimethylammonium chloride. More preferably, the dialkyldimethylammonium chloride is didecyldimethylammonium chloride or DDAC having the following chemical structure:

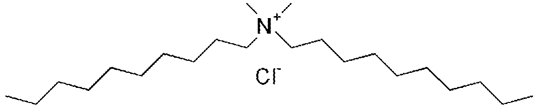

Dialkyldimethylammonium chlorides are commercially available, for example as Acticide^{®} from Thor, and Varisoft^{®} from Evonik Industries. DDAC is also available from Sigma Aldrich.

The disinfectant composition of the invention includes the three components (i), (ii) and (iii) at defined concentrations. The use of the invention similarly includes HT61 or a pharmaceutically acceptable salt and/or solvate thereof at a defined concentration. A suitable concentration for 4-methyl-8-phenoxy-1-(2-phenylethyl)-2,3-dihydro-1H-pyrrolo[3,2-c]quinoline or a pharmaceutically acceptable derivative thereof is from about 0.001 to about 0.5% w/v, based on the whole composition. Preferably from about 0.001 to about 0.4% w/v, more preferably from about 0.001 to about 0.2 % w/v, and particularly preferably from about 0.01 to about 0.1% w/v, for example 0.001, 0.0025, 0.005, 0.075, 0.01, 0.02, 0.03, 0.04, 0.05, 0.06, 0.07, 0.08, 0.09, 0.1, 0.2, 0.3, 0.4 or 0.5 % w/v of the disinfectant composition.

In various embodiments, the concentration of 4-methyl-8-phenoxy-1-(2-phenylethyl)-2,3-dihydro-1H-pyrrolo[3,2-c]quinoline or a pharmaceutically acceptable derivative thereof in the composition of the invention is from about 0.001 to about 0.5 % w/v, preferably from about 0.001 to about 0.4 % w/v, more preferably from about 0.001 to about 0.25 % w/v.

A suitable concentration for the chlorhexidine or a pharmaceutically acceptable salt and/or solvate thereof is about 0.001 to about 4% w/v, preferably 0.001 to about 3% w/v, more preferably 0.001% to about 2% w/v, particularly preferably from about 0.05 to about 0.5% w/v, based on the disinfectant composition as a whole. For example 0.001, 0.0025, 0.005, 0.0075, 0.01, 0.025, 0.05, 0.075, 0.1, 0.2, 0.3, 0.4, 0.5, 0.6, 0.7, 0.8, 0.9, 1.0, 1.5, 2.0, 3.0, or 4.0% w/v of the composition.

In various embodiments of the present invention, the concentration of chlorhexidine is less than about 2% w/v, for example 0.01 to 2% w/v, preferably 0.01 to 1.5% w/v and more preferably 0.01 to 1.0% w/v.

The percentage w/v (% w/v) of chlorhexidine or a pharmaceutically acceptable salt and/or solvate thereof is calculated based on the weight of the chlorhexidine or chlorhexidine salt *per se* in the composition. For example, "0.1% w/v chlorhexidine digluconate" means that there is 0.1% of the chlorhexidine digluconate in the composition based on the weight of the salt and the volume of the overall composition.

A suitable concentration for the dialkyldimethylammonium chloride (e.g. DDAC) is from about 0.001 to about 2% w/v, preferably from about 0.001 to 1% w/v, more preferably from about 0.01 to 0.7% w/v, based on the whole disinfectant composition. For example, 0.001, 0.0025, 0.005, 0.0075, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.25, 1.5, 2.0% w/v of the composition.

In one embodiment the disinfectant composition comprises about 0.005 to about 0.5 % w/v of 4-methyl-8-phenoxy-1-(2-phenylethyl)-2,3-dihydro-1H-pyrrolo[3,2-c]quinoline or a pharmaceutically acceptable salt and/or solvate thereof, about 0.01 % w/v to about 1.0% w/v of chlorhexidine or a pharmaceutically acceptable salt and/or solvate thereof, and about 0.01% w/v to about 1.0% w/v of a dialkyldimethylammonium chloride.

In one embodiment the disinfectant composition comprises about 0.005 to about 0.5 % w/v of 4-methyl-8-phenoxy-1-(2-phenylethyl)-2,3-dihydro-1H-pyrrolo[3,2-c]quinoline or a pharmaceutically acceptable salt and/or solvate thereof, about 0.05 % w/v to about 0.5% w/v of chlorhexidine or a pharmaceutically acceptable salt and/or solvate thereof, and about 0.01% w/v to about 1.0% w/v of a dialkyldimethylammonium chloride.

In one embodiment the disinfectant composition comprises about 0.005 to about 0.5 % w/v of 4-methyl-8-phenoxy-1-(2-phenylethyl)-2,3-dihydro-1H-pyrrolo[3,2-c]quinoline or a pharmaceutically acceptable salt and/or solvate thereof, about 0.1% w/v to about 0.5% w/v of chlorhexidine or a pharmaceutically acceptable salt and/or solvate thereof, and about 0.01% w/v to about 1.0% w/v of a dialkyldimethylammonium chloride.

In one embodiment the disinfectant composition comprises about 0.005 to about 0.2 % w/v of 4-methyl-8-phenoxy-1-(2-phenylethyl)-2,3-dihydro-1H-pyrrolo[3,2-c]quinoline or a pharmaceutically acceptable salt and/or solvate thereof, about 0.01 % w/v to about 1.0% w/v of chlorhexidine or a pharmaceutically acceptable salt and/or solvate thereof, and about 0.01% w/v to about 1.0% w/v of a dialkyldimethylammonium chloride.

In one embodiment the disinfectant composition comprises about 0.005 to about 0.2 % w/v of 4-methyl-8-phenoxy-1-(2-phenylethyl)-2,3-dihydro-1H-pyrrolo[3,2-c]quinoline or a pharmaceutically acceptable salt and/or solvate thereof, about 0.01 % w/v to about 0.5% w/v of chlorhexidine or a pharmaceutically acceptable salt and/or solvate thereof, and about 0.01% w/v to about 1.0% w/v of a dialkyldimethylammonium chloride.

The weight ratio of the components (i), (ii) and (iii) in the disinfectant composition of the present invention is not limited provided that each component is included at the requisite concentration range disclosed herein. In various embodiments, however, the chlorhexidine and DDAC components are included at a weight ratio of from about 0.05:1 to about 1:1, preferably from about 0.1:1 to about 1:1, more preferably from about 0.1:1 to about 0.7:1. Additionally or alternatively, HT61 and chlorhexidine may be included at a weight ratio of from about 0.05:1 to about 1:1, preferably from about 0.05:1 to about 0.5:1, more preferably from about 0.1:1 to about 0.3:1. Additionally or alternatively, the weight ratio of HT61:chlorhexidine:DDAC may be from about 0.05:1:10 to about 0.5:1:1, preferably from about 0.05:1:8 to about 0.5:1:1, more preferably from about 0.05:1:7 to about 0.3:1:1.

The disinfectant composition of the present invention is formulated as a single solution in an aqueous solvent. By the expression "formulated as a single solution" is meant that the disinfectant composition does not have to be mixed prior to use. This can facilitate ease of use and reflects the compatibility of the components in the aqueous solvent. The expression does not, however, exclude dilution of the disinfectant composition as in various embodiments the composition may be prepared as a concentrate and then diluted to a desired concentration. When prepared as a concentrate, the concentrations of the various components may of course be higher than noted above. For example, the DDAC may be included at a concentration of about 0.01 to about 20% w/v when included in a concentrate. Preferably from about 0.05 to about 10% w/v, more preferably from about 0.1 to about 5% w/v.

In one embodiment the disinfectant composition comprises about 0.005 to about 0.5 % w/v of 4-methyl-8-phenoxy-1-(2-phenylethyl)-2,3-dihydro-1H-pyrrolo[3,2-c]quinoline or a pharmaceutically acceptable salt and/or solvate thereof, about 0.01 % w/v to about 2.0% w/v of chlorhexidine or a pharmaceutically acceptable salt and/or solvate thereof, and about 0.01% w/v to about 5.0% w/v of a dialkyldimethylammonium chloride.

The aqueous solvent used in the present invention is not limited and includes any known aqueous solvents in the art. In various embodiments, the disinfectant composition includes at least 50 wt% water, preferably at least 70 wt% water and more preferably at least 90 wt%. These wt% water may be combined with the concentrations set out above for each component (i), (ii) and/or (iii). In various embodiments, the aqueous solvent is water.

For the reasons presented above, the aqueous disinfectant composition of the invention may be free of alcohol. By alcohol is meant any organic compound, which has a hydroxyl functional group bonded to a saturated carbon atom. In various embodiments, the composition is free from C1-8 alcohols, for example: methanol, ethanol, propanol, butanol, pentanol, hexanol and isomers and mixtures thereof. In one or more embodiments, the composition is free from ethanol, propanol, butanol or isomers or mixtures thereof.

In various embodiments of the invention, the composition includes one or more additional skin-conditioners or emollients. Non-limiting examples include aloe, vitamin E, vitamin E acetate, Vitamin B3, C6-10 alkane diols, coco-caprylate lactic acid and urea. The composition may, for example, include an amount of from 0.0001 to about 1.0 wt% of one or more additional skin-conditioners or emollient. In other embodiments, the composition is free from additional skin-conditioners and emollients.

In various embodiments of the invention, the composition additionally contains non-aqueous solvents, for example, selected from the group of alkanolamines, glycols or glycol ethers, or alcohols provided that they are miscible with water in the concentration range given so that the aqueous disinfectant composition can still be formed as a single solution. The non-aqueous solvents may be selected from ethylene glycol, propylene glycol, glycerol, 1,3-butanediol, glycerol, ethylene glycol methyl ether, ethylene glycol ethyl ether, ethylene glycol propyl ether, ethylene glycol mono-n-butyl ether, diethylene glycol methyl ether, diethylene glycol ethyl ether, propylene glycol methyl, ethyl or propyl ether, dipropylene glycol monomethyl or ethyl ether, di-isopropylene glycol monomethyl or ethyl ether, propylene glycol t-butyl ether and mixtures of these solvents. In various embodiments of the invention, the non-aqueous solvents may be selected from ethylene glycol methyl ether, ethylene glycol ethyl ether, ethylene glycol propyl ether, ethylene glycol mono-n-butyl ether, diethylene glycol methyl ether, diethylene glycol ethyl ether, propylene glycol methyl, ethyl or propyl ether, dipropylene glycol monomethyl or ethyl ether, di-isopropylene glycol monomethyl or ethyl ether, propylene glycol t-butyl ether and mixtures of these solvents. In various embodiments, the non-aqueous solvent comprises glycerol. Non-aqueous solvents, if present, are used in quantities between about 0.5 and about 15 wt%, preferably less than about 9 wt%, such as about 0.5 wt% to 9 wt%, more preferably less than about 5 wt%, such as about 0.5 wt% to about 5 wt%. In various embodiments, the disinfectant composition is free from non-aqueous solvents.

In various embodiments the aqueous disinfectant composition of the invention can be comprised in a cleaning agent and said cleaning agent or the disinfectant composition itself can additionally comprise one or more components that are commonly used in cleaning agents, for example those selected from the group consisting of perfumes, dyes, preservatives, surfactants, pH modifiers and the like. The disinfectant composition may, for example, be formulated as a concentrate and then diluted with water to prepare a cleaning agent. The cleaning agent may also be considered a disinfectant composition according to the present invention since it will typically include components (i), (ii) and (iii) in the requisite concentration ranges and be formulated as a single solution in an aqueous solvent.

The quaternary ammonium salt, dialkyldimethylammonium chloride, is preferably a cationic surfactant. Still, in addition thereto, various embodiments include additional surfactants in the composition or cleaning agent, preferably non-ionic surfactants. The non-ionic surfactant may be selected from the group consisting of polyalkoxylates, alkoxylated fatty alcohols, alkoxylated fatty acids, alkoxylated alkylphenols and block copolymers of ethylene oxide and propylene oxide. In other embodiments, the composition or cleaning agent does not include a further surfactant.

A perfume composition may be included in a quantity of usually up to 2 wt%, preferably 0.01 to 1 wt%, more preferably 0.02 to 0.75 wt%. The perfume composition can contain individual fragrance compounds or mixtures thereof. Suitable compounds are known in the art and can include esters, ethers, aldehydes, ketones, alcohols and hydrocarbons. The perfume composition can also contain natural fragrance compounds as may be obtained from plant sources.

All known acids or bases may be used as pH adjusters and in this way can be used to adjust the pH in either direction (i.e. make the composition more acidic or more basic. To improve the aesthetic impression of the composition they can be coloured using suitable dyes.

Preferred dyes possess high storage stability and are not sensitive to the other ingredients of the composition or light.

In one embodiment of the invention, the disinfectant composition further comprises a betalactam antimicrobial agent as described in the Applicant's patent application WO 2012/017215. Suitable beta-lactams for use in the present invention include the following compounds:
(i) penicillins, such as
   (I) benzylpenicillin, procaine benzylpenicillin, phenoxy-methylpenicillin, methicillin, propicillin, epicillin, cyclacillin, hetacillin, 6-aminopenicillanic acid, penicillic acid, penicillanic acid sulphone (sulbactam), penicillin G, penicillin V, phenethicillin, phenoxymethylpenicillinic acid, azlocillin, carbenicillin, cloxacillin, D-(-)-penicillamine, dicloxacillin, nafcillin and oxacillin,
   (II) penicillinase-resistant penicillins (e.g. flucloxacillin),
   (III) broad-spectrum penicillins (e.g. ampicillin, amoxicillin, metampicillin and bacampicillin),
   (IV) antipseudomonal penicillins (e.g. carboxypenicillins such as ticarcillin or ureidopenicillins such as piperacillin),
   (V) mecillinams (e.g. pivmecillinam), or
   (VI) combinations of any two or more of the agents mentioned at (I) to (V) above, or combinations of any of the agents mentioned at (I) to (V) above with a β-lactamase inhibitor such as tazobactam or, particularly, clavulanic acid (which acid is optionally in metal salt form, e.g. in salt form with an alkali metal such as sodium or, particularly, potassium);
(ii) cephalosporins, such as cefaclor, cefadroxil, cefalexin (cephalexin), cefcapene, cefcapene pivoxil, cefdinir, cefditoren, cefditoren pivoxil, cefixime, cefotaxime, cefpirome, cefpodoxime, cefpodoxime proxetil, cefprozil, cefradine, ceftazidime, cefteram, cefteram pivoxil, ceftriaxone, cefuroxime, cefuroxime axetil, cephaloridine, cephacetrile, cephamandole, cephaloglycine, ceftobiprole, PPI-0903 (TAK-599), 7-aminocephalosporanic acid, 7-aminodes-acetoxycephalosporanic acid, cefamandole, cefazolin, cefmetazole, cefoperazone, cefsulodin, cephalosporin C zinc salt, cephalothin, cephapirin; and
(iii) other β-lactams, such as monobactams (e.g. aztreonam), carbapenems (e.g. imipenem (optionally in combination with a renal enzyme inhibitor such as cilastatin), meropenem, ertapenem, doripenem (S-4661) and RO4908463 (CS-023)), penems (e.g. faropenem) and 1-oxa-β-lactams (e.g. moxalactam).

Methods of producing disinfectant compositions are known in the art; such a method may, for example, involve combining the components and adding to the aqueous solvent (e.g. water). In various embodiments the disinfectant composition of the invention is suitable for topical use. This means that the composition is suitable for application directly to a surface, such as the surface of a human or animal body, including skin, and/or other surfaces, such as hair and nails. The disinfectant composition may therefore be in the form of a skin cleanser, skin sanitizer, skin protectant, a wipe, a salve, a gel etc.. A wide variety of vehicles may be used to deliver the composition, such as for example, pads, bandages, patches, sticks, aerosol dispersers, pump sprays, trigger sprays, canisters, foam pumps, wipes and the like. In various embodiments the invention therefore provides a consumer product comprising the detergent composition where the product comprises a disinfectant spray, hand sanitizer solution or gel, antiseptic wipe or patch.

In such embodiments the disinfectant composition may include components typically included for these applications. The composition may, for example, include a thickening agent (e.g. hydroxymethyl cellulose, hydroxypropyl cellulose, carboxymethyl cellulose or carbomer); or a gelling agent (e.g. a polyoxyethylene-polyoxypropylene copolymer); a preservative (e.g. benzyl alcohol, benzalkonium chloride, chlorhexidine, chlorbutol, a benzoate, potassium sorbate or EDTA or salt thereof).

The disinfectant composition may also be suitable for use on non-mammalian surfaces, that is, surfaces beside those on the human body. For example, the composition may be used to disinfect various surfaces in homes and buildings as well as on building materials and furniture. Furthermore, the disinfectant composition may be clinical-grade and/or may be used to disinfect objects as well as surfaces, such objects may include medical equipment including medical devices.

Accordingly the invention relates to the non-therapeutic use of the disinfectant composition or cleaning agent according to the invention for cleaning and/or disinfecting an article or surface and to a non-therapeutic method for disinfecting a surface or article. Preferably the surface is a hard surface and the use of the disinfectant composition or cleaning agent reduces the number of microorganisms on the article or surface treated therewith. The contacting or application of the composition or cleaning to the article or surface may be done by any suitable method, for example, by pouring, spraying or otherwise wetting the article or surface with the composition or agent of the invention. The composition may, for example, be applied by rubbing the article/surface with a brush, sponge or cloth wetted with the composition.

Embodiments of the disinfectant composition include those described in the Examples below and as follows:

| **Ingredient** | | | | | | | |
|---|---|---|---|---|---|---|---|
| HT61 | 0.10% | 0.01% | 0.05% | 0.05% | 0.05% | 0.05% | 0.05% |
| Chlorhexidine (e.g. chlorhexidine digluconate) | 1.00% | 0.10% | 0.50% | 0.50% | 0.25% | 0.25% | 0.50% |
| DDAC | 1.40% | 0.14% | 0.18% | 0.70% | 0.14% | 0.18% | 0.18% |
| Other additives (e.g. non-ionic surfactant) | 7.35% | 0.74% | 0.74% | 3.68% | 0.37% | 0.35% | 0.25% |
| Water | - | Balance | Balance | Balance | Balance | Balance | Balance |
| Concentrate/Ready-to-use | Concentrate | Ready-to-use | Ready-to-use | Ready-to-use | Ready-to-use | Ready-to-use | Ready-to-use |

The present disclosure will now be exemplified with reference to the following non-limiting examples.

### Examples

### Example 1: HT61 as an Emollient

To support the use of a low concentration of HT61 acting as an emollient in an aqueous disinfectant composition, the surface tension, contact angle and hydrophilic lipophilic balance (HLB) of this compound were determined.

| | |
|---|---|
| Sample Preparation: | A 0.03% w/w solution was required for both the contact angle and surface tension measurements. This solution was prepared by adding 0.075 g of the HT61 solid (obtained according to the method set out in WO 2007/054693 A1 discussed above) to 250 ml of demineralised water. Some solid remained in solution so analysis was carried out using UV-Vis Spectroscopy. This analysis confirmed two large absorption peaks in the region of 350-400 nm; these peaks were assigned to the compound and a conclusion reached that HT61 had dissolved such that the solution was saturated. |
| | |
| | A test sample of approximately 100 ml was decanted from the volumetric flask and used for the surface tension and contact angle measurements. |
| Methods: | The surface tension of the sample was measured using a DataPhysics DCAT 9 tensionmeter and the Wilhelmy plate method. The |
| | measurement was made three times, with the plate being cleaned between each measurement. |
| | |
| | The contact angle was measured on the surface of parafilm. A sample of parafilm was wrapped around a glass slide and the protective film removed. The contact angle of the liquid with the parafilm was measured by placing a small (e.g. 2 µl) drop of test liquid (the sample) on the test surface. The profile of the drop was measured as a function of time using a DataPhysics OCA 35. Each liquid was measured three times. The contact angles were determined 2s, 12s and 30s after the drop first touched the surface. For reference, the contact angle of demineralised water was also measured. |
| | |
| | The HLB Value was obtained using Grifin's mathematical method (Griffin, W.C., Calculation of HLB Values of Non-Ionic Surfactants, Journal of the Society of Cosmetic Chemists, 1954, 5(4), 249-56): |
| | |
| | HLB = 20 x (Mₕ/M), |
| | |
| | where Mₕ is the molecular weight of hydrophilic groups (44 gmol⁻¹) and M is the molecular weight of the whole molecule (380 gmol⁻¹). |
| | |
| | The factor 20 is a scaling factor. Thus a scale of 1 to 20 is generally obtained. |

### Results:

| **Sample** | **Average Surface Tension mN/m** | **Average Contact Angle @2s** | **Average Contact Angle @12s** | **Average Contact Angle @30s** |
|---|---|---|---|---|
| Demineralised water | 72.5 | 111 | 110 | 110 |
| HT61 solution | 55.7 | 98 | 96 | 96 |

The HLB value for HT61 using Grifin's mathematical method is 2.3.

### Conclusions:

Assuming that the HT61 solution was saturated, there is a noticeable difference in the surface tension of the demineralised water and that of the HT61 solution (55.7 vs 72.5 mN/m). The surface tension of an emollient is the elastic-like force between the emollient and air; it has a strong impact on the wetting properties of an emollient on a surface. Surface tension, together with viscosity, correlates with spreadability.

The contact angle of the HT61 solution was also lower than the value for demineralised water: 96 vs 110 for water at 30s. To further characterize spreadability, contact angle and spreading value can be measured. If the liquid molecules are strongly attracted to the solid molecules then the liquid drop will completely spread out on the solid surface, corresponding to a contact angle of 0°. This is often the case for water on bare metallic or ceramic surfaces, angle. Finally, the HLB Value for HT61 is 2.3; hydrophilic-lipophilic balance (HLB) is the balance of the size and strength of the hydrophilic and lipophilic moieties of a surfactant molecule. An HLB value of 1 indicates a lipophilic compound and a chemical compound with an HLB value of 20 has a high hydrophilic portion.

In summary, the surface tension, contact angle and HLB value for HT61 is supports its emollient properties and hence the use of this compound in the present invention.

### Example 2: Disinfectant composition preparation

The following disinfectant compositions were prepared, each of the values is %w/v unless otherwise indicated:

| **Sample No.** | **HT61** | **Chlorhexidine** | **DDAC** | **Other Additives** | **Solvent** |
|---|---|---|---|---|---|
| 1 | 0.01 | 0.10 | 0.14 | 5.00 | Water, Balance |
| 2 | 0.01 | 0.10 | 0.70 | 2.50 | Water, Balance |
| 3 | 0.05 | 0.50 | 0.70 | 2.50 | Water, Balance |
| 4 | 0.10 | 0.50 | 0.70 | 2.50 | Water, Balance |
| 5 | 0.10 | 0.50 | 0.70 | 2.50 | Water, Balance |

Sample 1 was prepared as a concentrate and diluted 1:10 to arrive at the ready-to-use form. All of the other samples were prepared in their ready-to-use form.

### Example 3: Activity Testing

The samples prepared in Example 2 were submitted for activity testing.

Initially two screen tests were carried out on each sample according to the BS EN 14476 protocol against Poliovirus and Vaccinia virus. Virucidal activity was observed.

The following sample was also submitted for activity testing according to the BS EN 14476 protocol. This sample is referred to as Blend G:

| **HT61** | **Chlorhexidine** | **DDAC** | **Other Additives (including non-ionic surfactant)** | **Solvent** |
|---|---|---|---|---|
| 0.05% | 0.50% | 0.18% | 0.25% | Balance |

Blend G is particularly useful as a "Rapid Hand Cleanser Fluid". The solvent is a mixture of non-aqueous and aqueous components, specifically 2.50% glycerol and 96.52% water.

The standard method BS EN 14476 describes a test method and the minimum requirements for virucidal activity of a chemical disinfectant and antiseptic products that form a homogeneous physically stable preparation when diluted with hard water - or in the case of ready to use products that are not diluted when applied - with water. Products can only be tested at a concentration of 80% as some dilution is always produced by adding the test organisms and interfering substances. This European Standard applies to products that are used in the medical area in the fields of hygienic handrub, hygienic handwash, instrument disinfection by immersion, surface disinfection by wiping, spraying, flooding or other means and textile disinfection.

This European Standard also applies to areas and situations where disinfection is medically indicated. Such indication occurs in patient care, for example: In hospitals, in community medical facilities and in dental institutions or in clinics of schools, of kindergartens and of nursing homes, and may occur in the workplace and in the home. It may also include services such as laundries and kitchens supplying products directly for patients.

### Outline of Test Method (Obligatory Test Conditions)

A sample of the test product is diluted in synthetic hard water in products diluted at point of use or water in the case of ready to use products is added to a test suspension of viruses in a solution of interfering substance. The mixture is maintained at one of the temperatures and contact times specified in the standard. At the end of this contact time, an aliquot is taken; the virucidal action in this portion is immediately suppressed by a validated method (dilutions of the sample in ice-cold cell maintenance medium). The dilutions are transferred into cell culture units either using monolayer or cell suspension. Infectivity tests are done either by plaque test or quantal tests. After incubation, the titres of infectivity are calculated according to Spearman and Käber or by plaque counting. Reduction of virus infectivity is calculated from differences of Ig virus titres before (virus control) and after treatment with the product.

### Acceptance Criteria

The product when tested as above shall demonstrate at least a 4log10 reduction against the test virus. The test is deemed valid where all control requirements are met.

The test information was specifically as follows:

| | |
|---|---|
| Storage Conditions | Ambient |
| Appearance of the Product | Clear Liquid |
| Neutralisation Method | Dilution |
| Product Diluent | Distilled water |
| Test Concentrations | Neat (80%), Mid-range (50%), Non active (0.1%) |
| Experimental Conditions | Clean |
| Interfering Substance | Clean 0.3 g/l Bovine Albumin |
| Test Temperature | 20°C ± 1°C |
| Temperature of Incubation | 37°C ± 1°C |
| Identification of the Viral Strains: | Modified vaccinia virus Ankara (MVA), ATCC VR-1508 |
| Contact Times | 1 minute ± 10s |
| Stability and Appearance During Test | No Change Observed (Homogeneous) |

Blend G achieved a 4-log reduction against Vaccinia virus, and thus had virucidal activity against to the BS EN 14476 protocol against Vaccinia virus.

Simultaneously with the screen tests, a dermatological patch test was carried out on each of the five samples of Example 2. This patch test did not identify any dermatological issues.

At least one of the samples was further tested against feline Coronavirus according to the BS EN 14476 protocol and for antibacterial activity according to the BS EN 1500 protocol. Virucidal activity and antibacterial activity was observed. Finally, at least one of the samples was tested according to the full BS EN 17126 protocol (sporicidal) including *C.difficile.* Sporicidal activity was observed.

Blend G was tested further for antibacterial activity according to the BS EN 1500 protocol. The experimental conditions were as outlined in the standard. Namely:

| | |
|---|---|
| Neutraliser: | BU Broth |
| Bacterial strains: | *E.coli* K12 NCTC 10538 |
| Product Dilution: | Neat (as supplied) |
| Test Product Application: | 4 ml applied to dry hands. Hands rubbed for 60 seconds |
| Incubation Temperature: | 36°C ± 1°C |

Blend G was not inferior to the reference product (Handrub containing 60% propan-2-ol) and hence met the requirements of EN 1500:2013.

### Example 4 - Further Accreditations

Additional blends were prepared and tested as follows.

### Blend A Disinfecting Cleaner Concentrate

| | |
|---|---|
| Chlorhexidine Digluconate (Chlorhex) | 1.00% |
| Didecyldimethyl Ammonium Chloride (DDAC) | 1.40% |
| HT61 | 0.10% |
| Other additives (e.g. non-ionic surfactant) | 7.35% |

### Blend B Disinfecting Cleaner Ready-to-use

| | |
|---|---|
| Chlorhex | 0.10% |
| DDAC | 0.14% |
| HT61 | 0.01% |
| Other additives (e.g. non-ionic surfactant) | 0.74% |
| Solvent | Balance |

### Blend C Disinfecting Cleaner (Clinical grade)

| | |
|---|---|
| Chlorhex | 0.50% |
| DDAC | 0.18% |
| HT61 | 0.05% |
| Other additives(e.g. non-ionic surfactant) | 0.74% |
| Solvent | Balance |

### Blend D Medical Device Disinfectant Cleaner

| | |
|---|---|
| Chlorhex | 0.50% |
| DDAC | 0.70% |
| HT61 | 0.05% |
| Other additives (e.g. non-ionic surfactant) | 3.68% |
| Balance | Solvent |

### Blend E Electro-static Sanitising Fluid

| | |
|---|---|
| Chlorhex | 0.25% |
| DDAC | 0.14% |
| HT61 | 0.05% |
| Other additives (e.g. non-ionic surfactant) | 0.37% |
| Solvent | Balance |

### Blend F Hand Sanitising Fluid

| | |
|---|---|
| Chlorhex | 0.25% |
| DDAC | 0.18% |
| HT61 | 0.05% |
| Other additives (e.g. non-ionic surfactant) | 0.35% |
| Solvent | Balance |

### Blends A & B

Exactly equivalent blends but with HT61 absent have achieved accreditations to BS EN 1276, 13697, 16615, 1560, 13624 and 13727. Accordingly the blends with HT61 will also achieve these accreditations. In addition, the presence of HT61 provides emollient properties as explained and supported by Example 1 above.

### Blend C

This blend is accredited to BS EN 1500 and 14476. Due to its increased Chlorhex and DDAC content it can be assumed to meet the requirements of the EN standards listed above for blends A & B.

### Blend D

This blend has been subjected to empirical tests in accordance with the methodology in ISO CEN ISO/TS15883-5:2005 Washer disinfections - Part 5. The test indicated a total removal of bio-film in a 10 second exposure time.

### Blend E

Feedback on ATP testing of this blend in 20 individual installations has indicated excellent sanitising results.

### Blend F

This blend is expected to meet the requirements of BS EN 1500 and 14476 in addition to the accreditations listed above for blends A & B.

### Blend G

This blend (see Example 3) has met the requirements of BS EN 1500 and 14,476. Due to its increased Chlorhex and DDAC content it can be assumed to meet the requirements of the EN standards listed above for blends A & B.

## Claims

1. Use of 0.001% w/v to 0.5% w/v of 4-methyl-8-phenoxy-1-(2-phenylethyl)-2,3-dihydro-1H-pyrrolo[3,2-c]quinoline or a pharmaceutically acceptable salt and/or solvate thereof as an emollient in an aqueous disinfectant composition.

2. Use of claim 1, wherein the disinfectant composition is a virucidal, antibacterial and/or sporicidal disinfectant composition.

3. A disinfectant composition comprising:
(i) 0.001% w/v to 0.5% w/v of 4-methyl-8-phenoxy-1-(2-phenylethyl)-2,3-dihydro-1H-pyrrolo[3,2-c]quinoline or a pharmaceutically acceptable salt and/or solvate thereof;
(ii) 0.001% w/v to 4% w/v of chlorhexidine or a pharmaceutically acceptable salt and/or solvate thereof; and
(iii) 0.001% w/v to 2 % w/v of a dialkyldimethylammonium chloride, wherein the alkyl group has 8 or more carbon atoms.
wherein the composition is formulated as a single solution in an aqueous solvent.

4. The disinfectant composition of claim 3, wherein the composition comprises 0.001% w/v to 0.2% w/v of 4-methyl-8-phenoxy-1-(2-phenylethyl)-2,3-dihydro-1H-pyrrolo[3,2-c]quinoline or a pharmaceutically acceptable salt and/or solvate thereof.

5. The disinfectant composition of claim 3 or claim 4, wherein the composition comprises 0.01% w/v to 0.1% w/v of 4-methyl-8-phenoxy-1-(2-phenylethyl)-2,3-dihydro-1H-pyrrolo[3,2-c]quinoline or a pharmaceutically acceptable salt and/or solvate thereof.

6. The disinfectant composition of any preceding claim 3 to 5, wherein the composition comprises 0.001% w/v to 2% w/v chlorhexidine or a pharmaceutically acceptable salt and/or solvate thereof.

7. The disinfectant composition of any preceding claim 3 to 6, wherein the composition comprises 0.05% w/v to 0.5% w/v chlorhexidine or a pharmaceutically acceptable salt and/or solvate thereof.

8. The disinfectant composition of any preceding claim 3 to 7, wherein the dialkyldimethylammonium chloride is selected from dioctyldimethylammonium chloride, octyldecyldimethylammonium chloride, and didecyldimethylammonium chloride, preferably wherein the dialkyldimethylammonium chloride is didecyldimethylammonium chloride.

9. The disinfectant composition of claim 8, wherein the composition comprises 0.001% w/v to 1% w/v of didecyldimethylammonium chloride, preferably 0.01% w/v to 0.7% w/v of didecyldimethylammonium chloride.

10. The disinfectant composition of any preceding claim 3 to 9, wherein the composition is biodegradeable.

11. The disinfectant composition of any preceding claim 3 to 10, wherein the aqueous solvent is water.

12. A consumer product comprising the disinfectant composition of any preceding claim 3 to 11.

13. The consumer product of claim 12, wherein the product comprises at least one of a disinfectant spray, hand sanitizer solution or gel, antiseptic wipe or antiseptic patch.

14. A non-therapeutic method of disinfecting an article or surface comprising treating said article or surface with the disinfectant composition of any preceding claim 3 to 11 or the consumer product of claim 12 or claim 13.

## Patentansprüche

1. Verwendung von 0,001 % w/v bis 0,5 % w/v 4-Methyl-8-phenoxy-1-(2-phenylethyl)-2,3-dihydro-1H-pyrrolo[3,2-c]chinolin oder eines pharmazeutisch unbedenklichen Salzes und/oder Solvats davon als Erweichungsmittel in einer wässrigen Desinfektionsmittelzusammensetzung.

2. Verwendung nach Anspruch 1, wobei die Desinfektionsmittelzusammensetzung eine viruzide, antibakterielle und/oder sporizide Desinfektionsmittelzusammensetzung ist.

3. Desinfektionsmittelzusammensetzung, umfassend:
(i) 0,001 % w/v bis 0,5 % w/v 4-Methyl-8-phenoxy-1-(2-phenylethyl)-2,3-dihydro-1H-pyrrolo[3,2-c]chinolin oder eines pharmazeutisch unbedenklichen Salzes und/oder Solvats davon;
(ii) 0,001 % w/v bis 4 % w/v Chlorhexidin oder eines pharmazeutisch unbedenklichen Salzes und/oder Solvats davon; und
(iii) 0,001 % w/v bis 2 % w/v eines Dialkyldimethylammoniumchlorids, wobei die Alkylgruppe 8 oder mehr Kohlenstoffatome aufweist;
wobei die Zusammensetzung als einzelne Lösung in einem wässrigen Lösungsmittel formuliert ist.

4. Desinfektionsmittelzusammensetzung nach Anspruch 3, wobei die Zusammensetzung 0,001 % w/v bis 0,2 % w/v 4-Methyl-8-phenoxy-1-(2-phenylethyl)-2,3-dihydro-1H-pyrrolo[3,2-c]chinolin oder eines pharmazeutisch unbedenklichen Salzes und/oder Solvats davon umfasst.

5. Desinfektionsmittelzusammensetzung nach Anspruch 3 oder Anspruch 4, wobei die Zusammensetzung 0,01% w/v bis 0,1% w/v 4-Methyl-8-phenoxy-1-(2-phenylethyl)-2,3-dihydro-1H-pyrrolo[3,2-c]chinolin oder eines pharmazeutisch unbedenklichen Salzes und/oder Solvats davon umfasst.

6. Desinfektionsmittelzusammensetzung nach einem der vorhergehenden Ansprüche 3 bis 5, wobei die Zusammensetzung 0,001 % w/v bis 2 % w/v Chlorhexidin oder eines pharmazeutisch unbedenklichen Salzes und/oder Solvats davon umfasst.

7. Desinfektionsmittelzusammensetzung nach einem der vorhergehenden Ansprüche 3 bis 6, wobei die Zusammensetzung 0,05 % w/v bis 0,5 % w/v Chlorhexidin oder eines pharmazeutisch unbedenklichen Salzes und/oder Solvats davon umfasst.

8. Desinfektionsmittelzusammensetzung nach einem der vorhergehenden Ansprüche 3 bis 7, wobei das Dialkyldimethylammoniumchlorid ausgewählt ist aus Dioctyldimethylammoniumchlorid, Octyldecyldimethylammoniumchlorid und Didecyldimethylammoniumchlorid, wobei das Dialkyldimethylammoniumchlorid bevorzugt Didecyldimethylammoniumchlorid ist.

9. Desinfektionsmittelzusammensetzung nach Anspruch 8, wobei die Zusammensetzung 0,001 % w/v bis 1 % w/v Didecyldimethylammoniumchlorid, bevorzugt 0,01 % w/v bis 0,7 % w/v Didecyldimethylammoniumchlorid umfasst.

10. Desinfektionsmittelzusammensetzung nach einem der vorhergehenden Ansprüche 3 bis 9, wobei die Zusammensetzung biologisch abbaubar ist.

11. Desinfektionsmittelzusammensetzung nach einem der vorhergehenden Ansprüche 3 bis 10, wobei das wässrige Lösungsmittel Wasser ist.

12. Verbraucherprodukt, umfassend die Desinfektionsmittelzusammensetzung nach einem der vorhergehenden Ansprüche 3 bis 11.

13. Verbraucherprodukt nach Anspruch 12, wobei das Produkt mindestens eines von einem Desinfektionsspray, einer Händedesinfektionslösung oder einem -gel, einem antiseptischen Tuch oder einem antiseptischen Pflaster umfasst.

14. Nichttherapeutisches Verfahren zum Desinfizieren eines Artikels oder einer Oberfläche, umfassend Behandeln des Artikels oder der Oberfläche mit der Desinfektionsmittelzusammensetzung nach einem der vorhergehenden Ansprüche 3 bis 11 oder dem Verbraucherprodukt nach Anspruch 12 oder Anspruch 13.

## Revendications

1. Utilisation de 0,001 % p/v à 0,5 % p/v de la 4-méthyl-8-phénoxy-1-(2-phényléthyl)-2,3-dihydro-1H-pyrrolo[3,2-c]quinoléine ou d'un sel et/ou solvate pharmaceutiquement acceptable(s) de celle-ci comme émollient dans une composition désinfectante aqueuse.

2. Utilisation de la revendication 1, dans laquelle la composition désinfectante est une composition désinfectante virucide, antibactérienne et/ou sporicide.

3. Composition désinfectante comprenant :
(i) 0,001 % p/v à 0,5 % p/v de la 4-méthyl-8-phénoxy-1-(2-phényléthyl)-2,3-dihydro-1H-pyrrolo[3,2-c]quinoléine ou d'un sel et/ou solvate pharmaceutiquement acceptable(s) de celle-ci ;
(ii) 0,001 % p/v à 4 % p/v de la chlorhexidine ou d'un sel et/ou solvate pharmaceutiquement acceptable(s) de celle-ci ; et
(iii) 0,001 % p/v à 2 % p/v d'un chlorure de dialkyldiméthylammonium, ledit groupe alkyle possède 8 atomes de carbone ou plus,
ladite composition étant formulée sous la forme d'une solution unique dans un solvant aqueux.

4. Composition désinfectante de la revendication 3, ladite composition comprenant 0,001 % p/v à 0,2 % p/v de la 4-méthyl-8-phénoxy-1-(2-phényléthyl)-2,3-dihydro-1H-pyrrolo[3,2-c]quinoléine ou d'un sel et/ou solvate pharmaceutiquement acceptable(s) de celle-ci.

5. Composition désinfectante de la revendication 3 ou la revendication 4, ladite composition comprenant 0,01 % p/v à 0,1 % p/v de la 4-méthyl-8-phénoxy-1-(2-phényléthyl)-2,3-dihydro-1H-pyrrolo[3,2-c]quinoléine ou d'un sel et/ou solvate pharmaceutiquement acceptable(s) de celle-ci.

6. Composition désinfectante de l'une quelconque des revendications précédentes 3 à 5, ladite composition comprenant 0,001 % p/v à 2 % p/v de la chlorhexidine ou d'un sel et/ou solvate pharmaceutiquement acceptable(s) de celle-ci.

7. Composition désinfectante de l'une quelconque des revendications précédentes 3 à 6, dans laquelle la composition comprend 0,05 % p/v à 0,5 % p/v de la chlorhexidine ou d'un sel et/ou solvate pharmaceutiquement acceptable(s) de celle-ci.

8. Composition désinfectante de l'une quelconque des revendications précédentes 3 à 7, dans laquelle le chlorure de dialkyldiméthylammonium est choisi parmi le chlorure de dioctyldiméthylammonium, le chlorure d' octyldécyldiméthylammonium et le chlorure de didécyldiméthylammonium, dans laquelle, de préférence, le chlorure de dialkyldiméthylammonium est le chlorure de didécyldiméthylammonium.

9. Composition désinfectante de la revendication 8, ladite composition comprenant 0,001 % p/v à 1 % p/v de chlorure de didécyldiméthylammonium, de préférence 0,01 % p/v à 0,7 % p/v de chlorure de didécyldiméthylammonium.

10. Composition désinfectante de l'une quelconque des revendications précédentes 3 à 9, dans laquelle la composition est biodégradable.

11. Composition désinfectante de l'une quelconque des revendications précédentes 3 à 10, dans laquelle le solvant aqueux est l'eau.

12. Produit de consommation comprenant la composition désinfectante de l'une quelconque des revendications précédentes 3 à 11.

13. Produit de consommation de la revendication 12, ledit produit comprenant au moins l'un d'un vaporisateur désinfectant, d'une solution ou d'un gel désinfectant pour les mains, d'une lingette antiseptique ou d'un timbre antiseptique.

14. Procédé non thérapeutique de désinfection d'un article ou d'une surface comprenant le traitement dudit article ou de ladite surface par la composition désinfectante de l'une quelconque des revendications précédentes 3 à 11 ou le produit de consommation de la revendication 12 ou la revendication 13.
